(19) Europäisches Patentamt
European Patent Office
Office européen des brevets

(11) **EP 4 421 687 A1**

(12) **EUROPEAN PATENT APPLICATION**
published in accordance with Art. 153(4) EPC

(43) Date of publication:
**28.08.2024 Bulletin 2024/35**

(21) Application number: **23821471.2**

(22) Date of filing: **22.05.2023**

(51) International Patent Classification (IPC):
*G06N 3/048* $^{(2023.01)}$   *C08L 101/00* $^{(2006.01)}$
*G06N 3/09* $^{(2023.01)}$   *G16C 20/70* $^{(2019.01)}$

(52) Cooperative Patent Classification (CPC):
**C08L 101/00; G06N 3/048; G06N 3/09; G16C 20/70**

(86) International application number:
**PCT/JP2023/019011**

(87) International publication number:
**WO 2024/142427 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.12.2022 JP 2022212704**

(71) Applicant: **DIC CORPORATION**
**Itabashi-ku**
**Tokyo 174-8520 (JP)**

(72) Inventor: **NAGAO Atsushi**
**Takaishi-shi, Osaka 5920001 (JP)**

(74) Representative: **TBK**
**Bavariaring 4-6**
**80336 München (DE)**

(54) **PREDICTION METHOD, INFORMATION PROCESSING DEVICE, AND PROGRAM**

(57)     To improve a predicting technology related to a predicting target. A method for prediction executed by an information processing device 10 includes a step of training a neural network model based on actual data related to a prediction target; and a step of predicting an objective factor related to the prediction target based on a plurality of explanatory factors related to the prediction target by the neural network model, in which the neural network model includes an input layer, an intermediate layer, and an output layer, and a coefficient of an activation function related to the intermediate layer is larger than a coefficient of an activation function related to the output layer.

FIG. 1

EP 4 421 687 A1

**Description**

Technical Field

The present disclosure relates to a method for prediction, an information processing device, and a program. The present application claims priority based on Japanese Patent Application No. 2022-212704 filed in Japan on December 28, 2022, and the content thereof is incorporated herein.

Background Art

Conventionally, methods for performing prediction related to physical and chemical phenomena such as chemical reactions have been developed (for example, PTL 1).

Citation List

Patent Literature

PTL 1: WO 2003/026791

Summary of Invention

Technical Problem

The technique described in PTL 1 has described the use of modeling techniques such as neural networks, partial least squares, and principal component regression to optimize the control of reactor systems. However, specific design methods and optimization of the neural network models in performing predictions have not been considered, and there has been room for improvement in the prediction technology related to prediction targets such as chemical reactions of synthetic resins.
An object of the present disclosure made in view of such circumstances is to improve the prediction technology related to the prediction targets.

Solution to Problem

(1) A method for prediction in one embodiment of the present disclosure is a method for prediction executed by an information processing device, the method comprising:

a step of training a neural network model based on actual data related to a prediction target; and
a step of predicting an objective factor related to the prediction target based on a plurality of explanatory factors related to the prediction target by the neural network model, in which
the neural network model comprises an input layer, an intermediate layer, and an output layer, and a coefficient of an activation function related to the intermediate layer is larger than a coefficient of an activation function related to the output layer.

(2) A method for prediction in one embodiment of the present disclosure is the method for prediction according to (1), in which

the activation functions related to the intermediate layer and the output layer are sigmoid functions determined by following Mathematical Formulas (1) and (2), respectively, and
$\alpha_1$ and $\alpha_2$ satisfy $\alpha_1 > \alpha_2$:

[Mathematical Formula 1]

$$f^1\left(u_j^1\right) = \frac{1}{1+e^{-a_1 u_j^1}} \quad (1)$$

$$f^2\left(u_j^2\right) = \frac{1}{1+e^{-a_2 u_j^2}} \quad (2)$$

with proviso that $f^1\left(u_j^1\right)$ is the activation function related to the intermediate layer; $\alpha_1$ is the coefficient of the activation function related to the intermediate layer; $u_j^1$ is an input value input to a j-th of the intermediate layer; $f^2\left(u_j^2\right)$ is the activation function related to the output layer; $\alpha_2$ is the coefficient of the activation function related to the output layer; and $u_j^2$ is an input value input to a j-th of the output layer.

(3) A method for prediction in one embodiment of the present disclosure is the method for prediction according to (1) or (2), in which the prediction target includes a polycondensation reaction and an addition polymerization reaction.
(4) A method for prediction in one embodiment of the present disclosure is the method for prediction according to any one of (1) to (3), in which the number of elements in the intermediate layer is 1.1 times or more and less than 6 times of the number of the explanatory factors.
(5) A method for prediction in one embodiment of the present disclosure is the method for prediction according to any one of (1) to (4), in which a numerical value range of the explanatory factors input to the input layer is 0 or more and 1 or less and a numerical value range of the objective factor output from the output layer is 0.2 or more and 0.8 or less.
(6) An information processing device in one embodiment of the present disclosure comprises a control unit, in which

the control unit

trains a neural network model based on actual data related to a prediction target, and
predicts an objective factor related to the prediction target based on a plurality of explanatory factors related to the prediction target by the neural network model, and

the neural network model comprises an input layer, an intermediate layer, and an output layer, and a coefficient of an activation function related to the intermediate layer is larger than a coefficient of an activation function related to the output layer.

(7) A non-transitory computer-readable recording medium in one embodiment of the present disclosure is a non-transitory computer-readable recording medium storing an instruction, when executed by a processor, the instruction causing the processor to:

train a neural network model based on actual data related to a prediction target; and
predict an objective factor related to the prediction target based on a plurality of explanatory factors related to the prediction target by the neural network model, in which
the neural network model comprises an input layer, an intermediate layer, and an output layer, and a coefficient of an activation function related to the intermediate layer is larger than a coefficient of an activation function related to the output layer.

Advantageous Effects of Invention

With the method for prediction, the information processing device, and the program in one embodiment of the present disclosure, the prediction technology related to the prediction target can be improved.

Brief Description of Drawings

FIG. 1 is a block diagram illustrating the schematic configuration of an information processing device that performs prediction related to chemical reactions of synthetic resins in the present embodiment.

FIG. 2 is a flowchart illustrating operations of the information processing device that performs prediction related to the chemical reactions of the synthetic resins in the present embodiment.

FIG. 3 is a conceptual diagram of a neural network model in the present embodiment.

FIG. 4 is a graph illustrating learning convergence results of the neural network model in the present embodiment.

FIG. 5 is a graph illustrating learning convergence results of the neural network model related to Comparative Example.

FIG. 6 is a graph illustrating learning convergence results of the neural network model related to Comparative Example.

FIG. 7 is a graph illustrating learning convergence results of the neural network model related to Comparative Example.

Description of Embodiments

Hereinafter, the method for performing the prediction related to the prediction target in the embodiment of the present disclosure will be described with reference to the drawings. The prediction target according to the present embodiment includes chemical reactions of synthetic resins. Hereinafter, in the present embodiment, the case where the prediction target is a chemical reaction of a synthetic resin will be described as one example. Here, the chemical reaction of the synthetic resin includes a polycondensation reaction and an addition polymerization reaction. Examples of the main polymer materials synthesized by the polycondensation reactions include polyesters, polyamides, polyethylene terephthalate, urea resins, phenolic resins, silicone resins, alkyd resins, alkyd resin polyethers, polyglucosides, melamine resins, and polycarbonates. Examples of the main polymer materials synthesized by the addition polymerization reactions include poly(meth)acrylic acid esters, polyethylene, polypropylene, polystyrene, polyvinyl chloride, polyvinyl acetate, polyvinylidene chloride, polyacrylonitrile, and polytetrafluoroethylene.

In each of the drawings, identical or equivalent parts are assigned the same symbols. In the description of the present embodiment, descriptions of the identical or equivalent parts are omitted or simplified as appropriate.

First, the overview of the present embodiment will be described. In the method for performing the prediction related to the chemical reaction of the synthetic resin in the present embodiment, a neural network model is trained based on actual data related to the chemical reaction of the synthetic resin. The trained neural network model is used to predict an objective factor related to the chemical reaction of the synthetic resin based on a plurality of explanatory factors related to the chemical reaction of the synthetic resin. Here, the neural network model according to the present embodiment includes an input layer, an intermediate layer, and an output layer, and the coefficient of an activation function related to the intermediate layer is larger than the coefficient of an activation function related to the output layer.

Therefore, the present embodiment is characterized in that the neural network model includes the input layer, the intermediate layer, and the output layer, and the coefficient of the activation function related to the intermediate layer is larger than the coefficient of the activation function related to the output layer. In the case where the prediction related to the chemical reaction of the synthetic resin is performed, the learning process can be optimized and the prediction accuracy can be improved by setting the coefficient of the activation function related to the intermediate layer larger than he coefficient of the activation function related to the output layer, as described later. Therefore, according the present embodiment, the prediction technology related to the chemical reaction of the synthetic resin can be improved.

(Configuration of Information Processing Device)

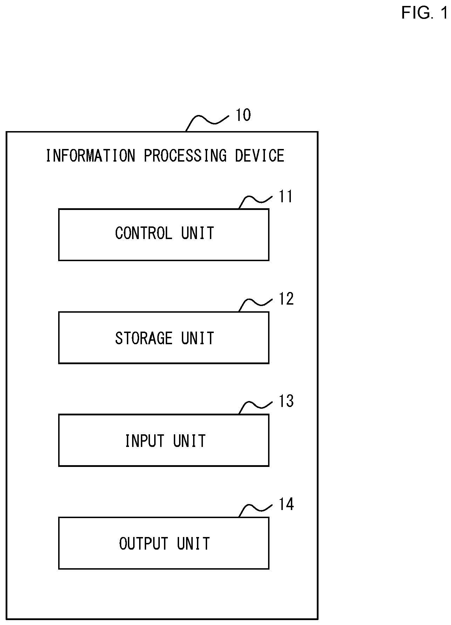

Subsequently, referring to FIG. 1, each configuration of an information processing device 10 will be described in detail. The information processing device 10 is an arbitrary device used by users. For example, personal computers, server computers, general-purpose electronic devices, or dedicated electronic devices can be employed as the information processing device 10.

As illustrated in FIG. 1, the information processing device 10 includes a control unit 11, a storage unit 12, an input unit 13, and an output unit 14.

The control unit 11 includes at least one processor, at least one dedicated circuit, or a combination thereof. The processor is a general-purpose processor such as a central processing unit (CPU) or a graphics processing unit (GPU), or a dedicated processor specialized for specific processing. The dedicated circuit is, for example, a field-programmable gate array (FPGA) or an application specific integrated circuit (ASIC). The control unit 11 executes processes associated with the operation of the information processing device 10 while controlling each part of the information processing device 10.

The storage unit 12 includes at least one semiconductor memory, at least one magnetic memory, at least one optical memory, or a combination of at least two of these memories. The semiconductor memory is, for example, a random-access memory (RAM) or a read-only memory (ROM). The RAM is, for example, a static random-access memory (SRAM) or a dynamic random-access memory (DRAM). The ROM is, for example, an electrically erasable programmable read-only memory (EEPROM). The storage unit 12 functions, for example, as a main memory device, an auxiliary memory device, or a cache memory. In the storage unit 12, data used in the operation of the information processing device 10 and data obtained by the operation of the information processing device 10 are stored.

The input unit 13 includes at least one interface for input. The interface for input is, for example, physical keys, capacitive keys, pointing devices, or touch screens integrated with displays. The interface for input may be, for example, a microphone that accepts voice input or a camera that accepts gesture input. The input unit 13 accepts operations to input data used in the operation of the information processing device 10. The input unit 13 may be connected to the information processing device 10 as an external input device instead of being provided in the information processing device 10. For example, any method such as universal serial bus (USB), high-definition multimedia interface (HDMI) (registered trademark), or Bluetooth (registered trademark) can be used as the connection method.

The output unit 14 includes at least one interface for output. The interface for output is, for example, a display that outputs information in the form of images. The display is, for example, a liquid crystal display (LCD) or an electroluminescence (EL) display. The output unit 14 displays and outputs data obtained by the operation of the information processing device 10. The output unit 14 may be connected to the information processing device 10 as an external output device instead of being provided in the information processing device 10. For example, any method such as USB, HDMI (registered trademark), or Bluetooth (registered trademark) can be used as the connection method.

The functions of the information processing device 10 are achieved by executing program according to the present embodiment on a processor corresponding to the information processing device 10. In other words, the functions of the information processing device 10 are achieved by the software. The program causes the computer to function as the information processing device 10 by causing the computer to execute the operations of the information processing device 10. In other words, the computer functions as the information processing device 10 by executing the operation of the information processing device 10 in accordance with the program.

In the present embodiment, the program can be recorded on a computer-readable recording medium. The computer-readable recording media include non-transient computer-readable media, for example, magnetic recording devices, optical discs, magneto-optical recording media, or semiconductor memories. Program distribution is performed by, for example, selling, assigning, or lending removal recording media such as a digital versatile disc (DVD) or a compact disc read only memory (CD-ROM) on which the program is recorded. Program distribution may also be done by storing the program in a storage on an external server and transmitting the program from the external server to other computers. The program may also be provided as a program product.

A part of or all of the functions of the information processing device 10 may be achieved by a dedicated circuit corresponding to the control unit 11. In other words, a part of or all of the functions of the information processing device 10 may be achieved by hardware.

In the present embodiment, the storage unit 12 stores, for example, actual data and prediction models. The actual data and the prediction model may be stored in an external device separate from the information processing device 10. In this case, the information processing device 10 may be equipped with an interface for external communication. The interface for communication may be either interface of a wired communication or interface of wireless communication. In the case of the wired communication, the interface for communication is, for example, a LAN interface or USB. In the case of the interface for wireless communication, the interface for communication is, for example, an interface compliant with mobile communication standards such as LTE, 4G, or 5G, or an interface compliant with short-range wireless communication such as Bluetooth (registered trademark). The interface for communication can receive data used in the operation of the information processing device 10 and can transmit data obtained by the operation of the information processing device 10.

(Operation of Information Processing Device)

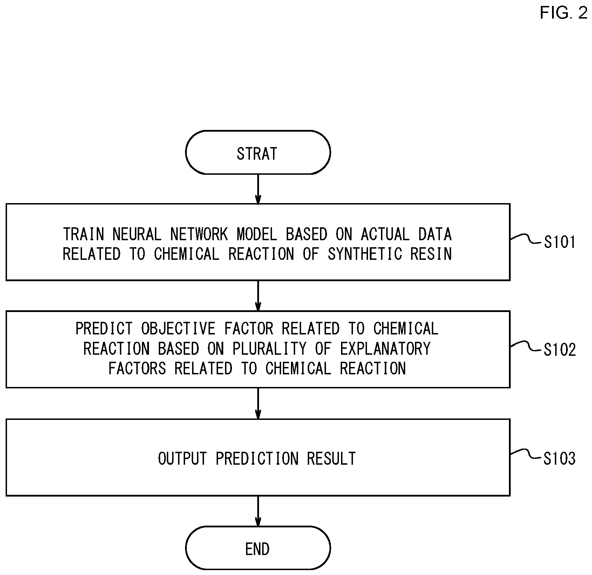

Subsequently, with reference to FIG. 2, the operation of the information processing device 10 according to the present embodiment will be described.

Step S101: The control unit 11 of the information processing device 10 trains a neural network model based on actual data related to the chemical reaction of the synthetic resin. The actual data includes the explanatory factors and the objective factor related to the chemical reaction of the synthetic resin. Such explanatory factors and objective factor are appropriately selected depending on the target chemical reaction of the synthetic resin to be predicted. The target chemical reaction of the synthetic resin to be predicted includes a polycondensation reaction and an addition polymerization reaction, for example. The polycondensation reaction includes a dehydration-condensation reaction. For example, in the case where the prediction related to the dehydration-condensation reaction is performed, the experimental data include the explanatory factors and the objective factor related to the dehydration-condensation reaction. For example, the explanatory factors may include feature values and the like related to the dehydration and temperature rising processes. The objective factor may also include a hydroxyl value, an acid value, and the like. In other words, the control unit 11 trains the neural network model using these explanatory factors and objective factor included in the actual data as the training data.

Any method can be employed to acquire the actual data. For example, the control unit 11 acquires the actual data from the storage unit 12. The control unit 11 may also acquire the actual data by accepting input of the actual data from the user by the input unit 13. Alternatively, the control unit 11 may acquire such actual data from an external device that stores the actual data through an interface for communication.

The neural network model trained based on the training data is cross-validated based on known data. As a result of such cross-validation, in the case where an accuracy is within a practical range, the prediction related to the chemical reaction of the synthetic resin is performed using the neural network model.

Step S102: The control unit 11 predicts the objective factor related to the chemical reaction of the synthetic resin based on the explanatory factors related to the chemical reaction of the synthetic resin. For example, the control unit 11 may acquire the objective factor by accepting input of the explanatory factors from the user by the input unit 13.

Step S103: The control unit 11 outputs the prediction result obtained in Step S102 using the output unit 14.

Here, the present embodiment is characterized in that the coefficients of the activation function are different between the intermediate layer and the output layer. Specifically, in the present embodiment, the coefficient of the activation function related to the intermediate layer is characterized by being larger than the coefficient of the activation function related to the output layer.

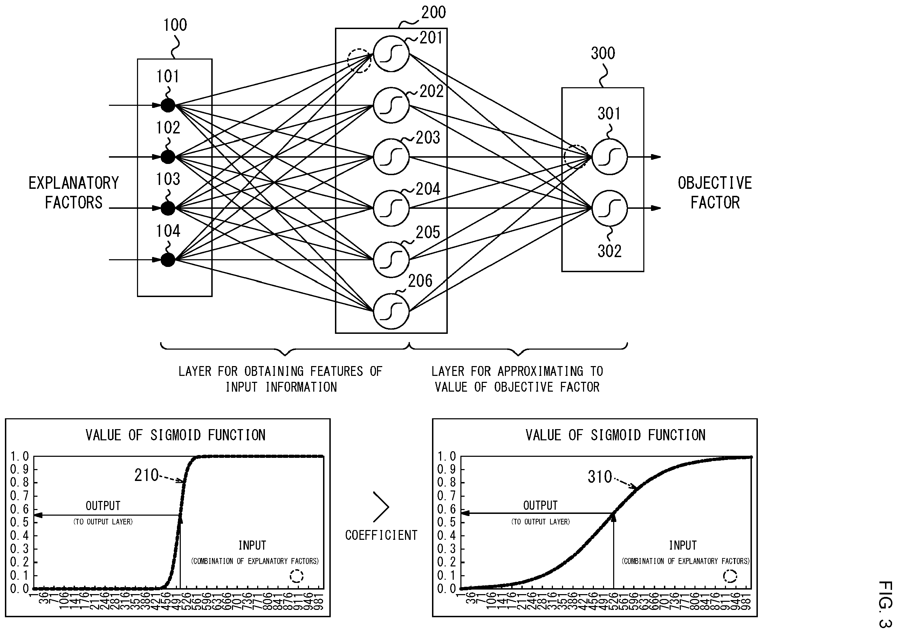

FIG. 3 is a conceptual diagram of the neural network model according to the present embodiment. The neural network model according to the present embodiment includes an input layer 100, an intermediate layer 200, and an output layer 300. The neural network model in the present embodiment is fully connected. In the present embodiment, the number of layers in the neural network model is, for example, 2. Such number of layers is the number of layers excluding the input layer. By setting the number of layers in the neural network model to 2, a model configuration can be prevented from becoming inappropriate to the physical phenomena in the chemical reaction of the synthetic resin. In other words, the number of layers of neural network model can be kept to minimum necessary, whereby the model configuration suitable for the physical phenomena in the chemical reaction of the synthetic resin can be achieved. The number of layers of the neural network model according to the present embodiment is not limited to this number of layers, and may be three layers or more. In the case where the number of layers in the neural network model is three or more, as the layer of the neural network model becomes more front side, the coefficient of the activation function may be set larger.

The input layer 100 includes a plurality of elements 101 to 104 (also referred to as input elements 101 to 104). In the neural network model illustrated in FIG. 3, the number of the input elements is 4. The input elements 101 to 104 are also referred to as the first to fourth elements, respectively. In the input elements 101 to 104, each of the explanatory factors is input. The number of the input elements, however, is not limited to this number.

The intermediate layer 200 includes a plurality of elements 201 to 206 (also referred to as intermediate elements 201 to 206). In the neural network model illustrated in FIG. 3, the number of the intermediate elements is 6. The intermediate elements 201 to 206 are also referred to as the first to sixth elements, respectively. The number of intermediate elements, however, is not limited to this number.

The output layer 300 includes a plurality of elements 301 and 302 (also referred to as output elements 301 and 302). In the neural network model illustrated in FIG. 3, the number of the output elements is 2. The output elements 301 and 302 are also referred to as the first and second elements, respectively. The number of the output elements, however, is not limited to this number.

The values input from the input elements 101 to 104 of the input layer 100 to the intermediate elements 201 to 206 of the intermediate layer 200 are converted in the intermediate layer 200 based on the activation function related to the intermediate layer 200. The converted values are output to the elements 301 and 302 of the output layer 300. The activation function related to the intermediate layer 200 is, for example, a sigmoid function. A graph 210 illustrates one example of the activation function related to the intermediate layer 200. The values input from the intermediate elements 201 to 206 of the intermediate layer 200 to the output elements 301 and 302 of the output layer 300 are converted in the output layer 300 based on the activation function related to output layer 300 and output. The activation function related to the output layer 300 is, for example, the sigmoid function. A graph 310 illustrates one example of the activation function related to the output layer 300. Specifically, the activation functions related to the intermediate layer and the output layer are, for example, the respective sigmoid functions determined by the following formulas (1) and (2).

[Mathematical Formula 2]

$$f^1(u_j^1) = \frac{1}{1+e^{-a_1 u_j^1}} \quad (1)$$

$$f^2(u_j^2) = \frac{1}{1+e^{-a_2 u_j^2}} \quad (2)$$

Here, $f^1(u_j^1)$ is the activation function related to the intermediate layer 200, $\alpha_1$ is the coefficient of the activation function related to the intermediate layer 200, and $u_j^1$ is the input value input to the j-th element of the intermediate layer 200. In the example in FIG. 3, the number of the intermediate elements is 6, and this j takes the value from 1 to 6.

$f^2(u_j^2)$ is the activation function relative to the output layer 300, $\alpha_2$ is the coefficient of the activation function related to the output layer 300, and $u_j^2$ is the input value input to the j-th element of the output layer 300. In the example in FIG. 3, j is 1 or 2 because the number of the output elements is 2. As described above, in the neural network model according to the present embodiment, the coefficient of the activation function related to the intermediate layer 200 is larger than the coefficient of the activation function related to the output layer 300. In other words, $\alpha_1$ and $\alpha_2$ in the neural network model according to the present embodiment are characterized by satisfying $\alpha_1 > \alpha_2$.

In the neural network model according to the present embodiment, the coefficient of the activation function related to the intermediate layer is larger than the coefficient of the activation function related to the output layer. This allows the configuration of the neural network model to be optimized at the time of performing the prediction related to the chemical reaction of the synthetic resin. Specifically, in the neural network model for performing the prediction related to the chemical reaction of the synthetic resin, change in the explanatory factors is desirably viewed as obvious change. Therefore, by setting the coefficient of the activation functions related to the intermediate layer larger than the coefficient of the activation functions related to the output layer, the change in the input values to the intermediate layer can be transmitted to the output layer as the obvious change. On the other hand, in the output layer of the neural network model for performing the prediction related to the chemical reaction of the synthetic resin, the values of the training data and the objective factor are required to be converged. Therefore, the coefficient of the activation function related to the output layer is set smaller than the coefficient of the activation function related to the intermediate layer. By doing so, the value of the objective factor output from the output layer is finely adjusted.

By setting the coefficients of the activation functions between the intermediate layer and the output layer to be different, the learning process of the neural network model is optimized. Specifically, the updated amount of the weight variables in the output layer and the intermediate layer during the learning process can be adjusted by changing the coefficient of the activation function. In addition, updating the weight variables provides a significant impact on the learning process. Therefore, the learning process may be optimized based on the adjustment of the updated amount. Hereinafter, the updated amount of the weight variables in an L-layer neural network model will be described.

First, the updated amount of the weight variables in the L-th layer (output layer) will be described. The updated amount of such weight variables is determined by Mathematical Formula (3) described below based on the partial derivative of the loss function.

[Mathematical Formula 3]

$$(Updated\ amount\ of\ weight\ variables\ in\ L-th\ layer) = -1 \times \varepsilon \times \frac{\partial E}{\partial w_{ij}^L} \quad (3)$$

Here, $\varepsilon$ is a learning constant, E is the loss function, and $w_{ij}^L$ is the weight variable between the i-th intermediate element of the L-1-th layer and the j-th element of the L-th layer of the neural network model.

The partial derivative of the loss function on the right-hand side of Mathematical Formula (3) can be transformed based on the chain rule of differentiation as follows.
[Mathematical Formula 4]

$$\frac{\partial E}{\partial w_{ij}^L} = \frac{\partial E}{\partial u_j^L}\frac{\partial u_j^L}{\partial w_{ij}^L} \quad (4)$$

Here, $u_j^L$ is the input value to the j-th element in the L-th layer of the neural network model. The neural network model in the present embodiment is fully connected. Therefore, $u_j^L$ is a weighted linear sum of values obtained by multiplying the output values of the intermediate elements in the L-1 layer of the neural network model by the respective weight variables wt.

When the first term on the right-hand side of Mathematical Formula (4) is defined as $\delta_j^L$, Mathematical Formula (4) can be transformed as follows.
[Mathematical Formula 5]

$$\frac{\partial E}{\partial u_j^L}\frac{\partial u_j^L}{\partial w_{ij}^L} = \delta_j^L z_i^{L-1} \quad (5)$$

Here, $z_i^{L-1}$ is the output value of the i-th intermediate element of the L-1-th layer of the neural network. The formula transformation of Mathematical Formula (5) is based on the relational formula of Mathematical Formula (6) below.
[Mathematical Formula 6]

$$\frac{\partial u_j^L}{\partial w_{ij}^L} = z_i^{L-1} \quad (6)$$

The first term $\delta_j^L$ of Mathematical Formula (5) can be transformed based on the chain rule of differentiation as follows.
[Mathematical Formula 7]

$$\delta_j^L = \frac{\partial E}{\partial z_j^L}\frac{\partial z_j^L}{\partial u_j^L} \quad (7)$$

Mathematical Formula (7) can be transformed as follows.
[Mathematical Formula 8]

$$\frac{\partial E}{\partial z_j^L}\frac{\partial z_j^L}{\partial u_j^L} = \frac{\partial E}{\partial y_j}\frac{\partial z_j^L}{\partial u_j^L} \quad (8)$$

Here, $y_j$ is the output value of the j-th element of the L-th layer (that is, the output layer) of the neural network, which is the same value as $z_j^L$.

In the case where the loss function is a squared error function, the loss function is represented as follows.
[Mathematical Formula 9]

$$E = \frac{1}{2}\Sigma_i(y_i - d_i)^2 \quad (9)$$

Here, $d_i$ is the i-th value of the training data.

When the loss function is the squared error function, Mathematical Formula (8) can be transformed as follows. [Mathematical Formula 10].

$$\frac{\partial E}{\partial y_j}\frac{\partial z_j^L}{\partial u_j^L} = (y_j - t_j)\frac{\partial z_j^L}{\partial u_j^L} \quad (10)$$

When the activation function of the L-th layer is the sigmoid function, the activation function is represented as follows.
[Mathematical Formula 11]

$$f^L\left(u_j^L\right) = \frac{1}{1+e^{-a^L u_j^L}} \quad (11)$$

Here, $f^L$ is the activation function of the L-th layer and $a^L$ is the coefficient of the sigmoid function of the L-th layer (output layer).

The second term on the right-hand side of Mathematical Formula (10) can be transformed based on Mathematical Formula (11) as follows.
[Mathematical Formula 12]

$$\frac{\partial z_j^L}{\partial u_j^L} = \frac{\partial f^L\left(u_j^L\right)}{\partial u_j^L} = a^L f^L\left(u_j^L\right)\left(1 - f^L\left(u_j^L\right)\right) \quad (12)$$

Mathematical Formula (12) can be further transformed as follows.
[Mathematical Formula 13]

$$a^L f^L\left(u_j^L\right)\left(1 - f^L\left(u_j^L\right)\right) = a^L z_j^L(1 - z_j^L) = a^L y_j(1 - y_j) \quad (13)$$

Based on Mathematical Formula (5) to Mathematical Formula (13), Mathematical Formula (4) can be transformed as follows.
[Mathematical Formula 14]

$$\frac{\partial E}{\partial w_{ij}^L} = (y_j - t_j)\frac{\partial z_j^L}{\partial u_j^L}z_i^{L-1} = a^L(y_j - t_j)(1 - y_j)y_j z_i^{L-1} \quad (14)$$

All of the variables on the right-hand side of Mathematical Formula (14) are values obtained by numerical calculation. Therefore, the value on the left-hand side can be determined by the numerical calculation. Therefore, the updated amount of each weight variable in the output layer represented by Mathematical Formula (3) can be obtained based on numerical calculations.

Subsequently, the updated amount of the intermediate layer in the learning process of the neural network model will be described. The updated amount of the weight variables of the intermediate layer is determined by the following Mathematical Formula (15) based on the partial derivative of the loss function.
[Mathematical Formula 15]

$$(Updated\ amount\ of\ weight\ variables\ in\ l-th\ layer) = -1 \times \varepsilon \times \frac{\partial E}{\partial w_{ij}^l} \quad (15)$$

Here, 1 represents the l-th layer of the neural network. In other words, the 1-th layer corresponds to the intermediate layer. is the weight variable between the i-th intermediate element of the l-1-th layer and the j-th element of the 1-th layer of the neural network.

The partial derivative of the loss function on the right-hand side of Mathematical Formula (15) can be transformed based on the chain rule of differentiation as follows.
[Mathematical Formula 16]

$$\frac{\partial E}{\partial w_{ij}^l} = \frac{\partial E}{\partial u_j^l}\frac{\partial u_j^l}{\partial w_{ij}^l} \quad (16)$$

Here, $u_j^l$ is the input value to the j-th element of the 1-th layer of the neural network. The neural network model in the present embodiment is fully connected. Therefore, $u_j^l$ is a weighted linear sum of values obtained by multiplying the output values of the intermediate elements in the l-1-th layer of the neural network by the respective weight variables $w_{ij}^l$.

When the first term on the right-hand side of Mathematical Formula (16) is defined as $\delta_j^l$, Mathematical Formula (16) can be transformed as follows.
[Mathematical Formula 17]

$$\frac{\partial E}{\partial u_j^l}\frac{\partial u_j^l}{\partial w_{ij}^l} = \delta_j^l z_i^{l-1} \quad (17)$$

Here, $z_i^{l-1}$ is the output value of the i-th intermediate element of the l-1-th layer of the neural network. The formula transformation of Mathematical Formula (17) is based on the relational formula of Mathematical Formula (18) below.
[Mathematical Formula 18]

$$\frac{\partial u_j^l}{\partial w_{ij}^l} = z_i^{l-1} \quad (18)$$

The first term of $\delta_j^l$ in Mathematical Formula (17) can be transformed based on the chain rule of differentiation as follows.
[Mathematical Formula 19]

$$\delta_j^l = \sum_k \frac{\partial E}{\partial u_k^{l+1}}\frac{\partial u_k^{l+1}}{\partial u_j^l} = \sum_k \delta_k^{l+1}\frac{\partial u_k^{l+1}}{\partial z_j^l}\frac{\partial z_j^l}{\partial u_j^l} \quad (19)$$

The right-hand side of Mathematical Formula (19) can be further transformed as follows.
[Mathematical Formula 20]

$$\sum_k \delta_k^{l+1}\frac{\partial u_k^{l+1}}{\partial z_j^l}\frac{\partial z_j^l}{\partial u_j^l} = \sum_k \delta_k^{l+1} w_{jk}^{l+1}\left\{f^l\left(u_j^l\right)\right\}' \quad (20)$$

When the activation function of the l-th layer is the sigmoid function, the activation function is represented as follows.
[Mathematical Formula 21]

$$f^l\left(u_j^l\right) = \frac{1}{1+e^{-a^l u_j^l}} \quad (21)$$

Here, $f^l$ is the activation function of the 1-th layer and $a^l$ is the coefficient of the sigmoid function of the 1-th layer.

When the activation function of the 1-th layer is the sigmoid function described above, the right-hand side of Mathematical Formula (20) can be transformed as follows.

[Mathematical Formula 22]

$$\sum_k \delta_k^{l+1} w_{jk}^{l+1} \{f^l(u_j^l)\}' = a^l f^l(u_j^l)\left(1 - f^l(u_j^l)\right)\sum_k \delta_k^{l+1} w_{jk}^{l+1} \quad (22)$$

Mathematical Formula (22) can be further transformed as follows.

[Mathematical Formula 23]

$$a^l f^l(u_j^l)\left(1 - f^l(u_j^l)\right)\sum_k \delta_k^{l+1} w_{jk}^{l+1} = a^l z_j^l\left(1 - z_j^l\right)\sum_k \delta_k^{l+1} w_{jk}^{l+1} \quad (23)$$

Based on Mathematical Formula (17) to Mathematical Formula (23), Mathematical Formula (16) can be transformed as follows.

[Mathematical Formula 24]

$$\frac{\partial E}{\partial w_{ij}^l} = \delta_j^l z_i^{l-1} = a^l z_j^l\left(1 - z_j^l\right)z_i^{l-1}\sum_k \delta_k^{l+1} w_{jk}^{l+1} \quad (24)$$

All of the variables on the right-hand side of Mathematical Formula (24) are values obtained by numerical calculation. Specifically, $\delta_k^{l+1}$ is a value sequentially determined from $\delta_j^l$ of the output layer. Other variables are values determined by numerical calculations. Therefore, the updated amount of each weight variable of the intermediate layer represented in Equation (15) can be obtained based on numerical calculations.

As represented in Mathematical Formula (14) and Mathematical Formula (24), the coefficients of the activation function are relevant in the process of calculating the updated amount of each weight variable. Specifically, when the activation functions of the intermediate layer and the output layer are the sigmoid functions, the respective updated amounts are proportional to $a^l$ and $a^L$ of the activation function. In other words, by changing the coefficients $a^l$ and $a^L$ of the sigmoid function, the updated amount of the weight variables can be adjusted and the learning process of the neural network model can be optimized.

Specifically, in the neural network model at the time of performing prediction related to the chemical reaction of synthetic resin, the updated amount of the weight variable related to the intermediate layer is preferably set to relatively large. This allows the weight variables in the intermediate layer to vary more significantly during the learning process, and thus changes in the input values to the intermediate layer to be transferred to the output layer as obvious changes. On the other hand, the updated amount of weight variables related to the output layer is preferably set to relatively small. This allows the weight variables in the output layer to vary less during the learning process and thus the values of the training data and the objective factor to be easily converged. In addition, by satisfying $a^l > a^L$, an arbitrary smooth function can be approximated with sufficient accuracy, eliminating the need to inadvertently increase the number of intermediate layers. This allows sufficient accuracy to be obtained even when the intermediate layer is one layer. Preparing fewer intermediate layers directly leads to reduction in generation of over-fitting and thus provides a secondary effect on stability of the learning process and, in addition, robustness of the model.

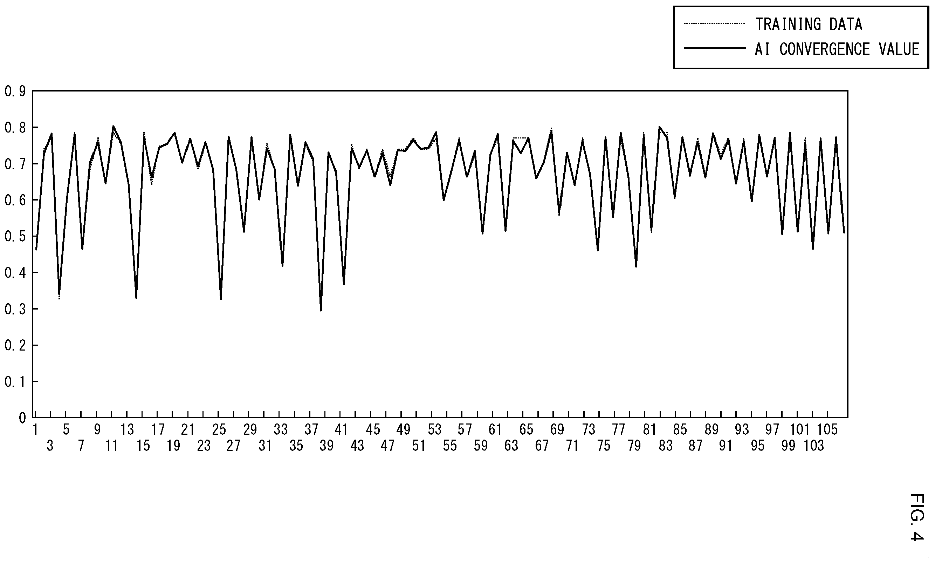

In the neural network model according to the present embodiment, the coefficient of the sigmoid function in the intermediate layer is, for example, 0.75 and the coefficient of the sigmoid function in the output layer is, for example, 0.1. FIG. 4 illustrates the learning convergence result of the neural network model of the prediction related to the chemical reaction of the synthetic resin when the coefficient of the sigmoid function in the intermediate layer is set to 0.75 and the coefficient of the sigmoid function in the output layer is set to 0.1. The training termination error is 0.003, the number of training cycles is 200,000, the number of the input factors is 13, the number of the intermediate elements is 20, and the number of the output factors is 1. As illustrated in FIG. 4, the values of the training data and the AI convergence values are almost identical and converge almost to limit values. As described above, in the neural network model for performing prediction related to the chemical reaction of the synthetic resin, a highly accurate prediction model can be obtained by setting the coefficient of the sigmoid function in the intermediate layer larger than the coefficient of the sigmoid function in the output layer.

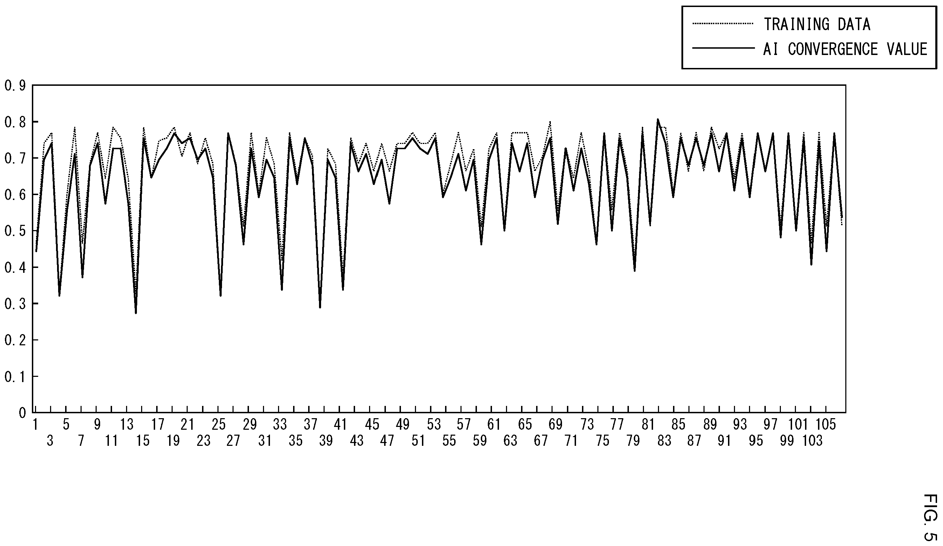

As Comparative Example, FIG. 5 illustrates the learning convergence result of the neural network model in the case where the coefficient of the sigmoid function in the intermediate layer is set to 0.75 and the coefficient of the sigmoid function in the output layer is set to 0.75. The training error, the number of the training cycles, the number of the input factors, the number of the intermediate elements, and the number of the output factors are the same as the training conditions in FIG. 4. As illustrated in FIG. 5, the values of the training data and the AI convergence values differ in some parts and are less accurate than those in the neural network model illustrated in FIG. 4.

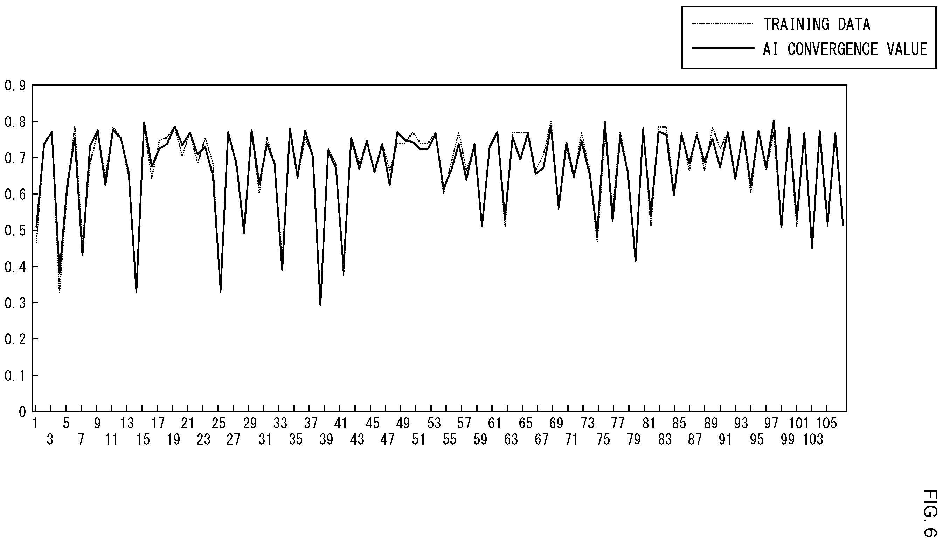

As Comparative Example, FIG. 6 illustrates the learning convergence result of the neural network model when the coefficient of the sigmoid function in the intermediate layer is set to 0.1 and the coefficient of the sigmoid function in the output layer is set to 0.1. The training error, the number of the training cycles, the number of the input factors, the number of the intermediate elements, and the number of the output factors are the same as the training conditions in FIG. 4. As illustrated in FIG. 6, the values of the training data and the AI convergence values differ in some parts and are less accurate than those in the neural network model illustrated in FIG. 4.

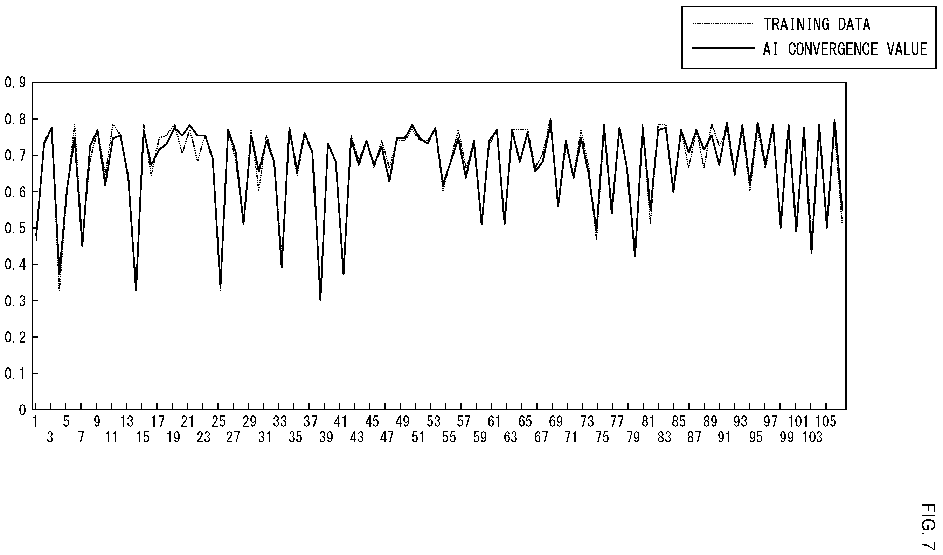

As Comparative Example, FIG. 7 illustrates the learning convergence result of the neural network model in the case where the coefficient of the sigmoid function in the intermediate layer is set to 0.4 and the coefficient of the sigmoid function in the output layer is set to 0.4. The training error, the number of the training cycles, the number of the input factors, the number of the intermediate elements, and the number of the output factors are the same as the training conditions in FIG. 4. As illustrated in FIG. 7, the values of the training data and the AI convergence values differ in some parts and are less accurate than those in the neural network model illustrated in FIG. 4.

As can be seen from the above learning convergence results of the models in FIGs. 4 to 7, the neural network model for predicting the chemical reaction of the synthetic resin has the highest accuracy when the coefficient of the sigmoid function in the intermediate layer is larger than the coefficient of the sigmoid function in the output layer. As described above, according to the present embodiment, the prediction technology for the chemical reaction of the synthetic resin can be improved.

In the present embodiment, the hyperparameters of the neural network model may be appropriately adjusted. For example, the learning constant may be any value as long as the learning constant is the smallest value at which the correction operation of the weights can be performed by differential operations. For example, in the present embodiment, the number of the intermediate elements of the neural network model may be 1.1 times or more and less than 6 times of the number of the explanatory factors (the number of the elements in the input layer). The number of the intermediate elements may be determined based on the number of the elements in the output layer. Specifically, for example, the number of the intermediate elements, the coefficient of the sigmoid function, and the number of training cycles can be adjusted as follows.

1. Number of intermediate elements

- The case where the number of output elements is 1: 1.1 times to 3 times
- The case where the number of output elements is 2: 1.1 times to 4.5 times
- The case where the number of output elements is 3: 1.1 times to 6 times

2. Coefficients of sigmoid function

Intermediate layer: 0.70 to 0.80
Output layer: 0.095 to 0.15

3. Number of training cycles
100,000 times to 200,000 times (the case where the training data is about 50 sets to about 100 sets)

In the present embodiment, the numerical range of each explanatory factor and the numerical range of each objective factor may be appropriately adjusted. For example, the numerical range of the explanatory factor input to the input layer may be 0 or more and 1 or less and the numerical range of the objective factor output from the output layer may be 0.2 or more and 0.8 or less. As described above, the explanatory factor side may be set to the full scale of 0 or more and 1 or less, which the neural network can handle. On the other hand, by limiting the objective factor side to 0.2 or more and 0.8 or less, the search range as numerical values can be narrowed to facilitate the search in numerical calculations.

The case where the activation functions of the intermediate layer and the output layer are sigmoid functions is described in the present embodiment. However, the activation functions are not limited to the sigmoid functions. For example, the activation functions of the intermediate layer and the output layer may be functions such as a hyperbolic tangent function (tanh function) and a ramp functions (ReLU).

In this embodiment, the case where the prediction target is the chemical reaction of the synthetic resin is described as one example. The prediction target, however, is not limited to this case. The prediction target may be, for example, the prediction of a physical or chemical phenomenon such as a chemical reaction of any substance. The prediction target may not necessarily be a physical or chemical phenomenon and the like. In other words, the technology according to the present embodiment can be used for the whole modeling using neural networks and the like.

Although the present disclosure has been described based on the drawings and examples, it should be noted that those skilled in the art can easily make changes and modifications based on the present disclosure. Therefore, it should be noted that these changes and modifications are included within the scope of the present disclosure. For example, the functions and the like included in the units, steps, or the like can be rearranged so as not to be logically inconsistent, and pluralities of units, steps, or the like can be combined to one or divided.

Reference Signs List

| 10 | information processing device |
| 11 | control unit |
| 12 | storage unit |
| 13 | input unit |
| 14 | output unit |
| 100 | input layer |
| 200 | intermediate layer |
| 300 | output layer |
| 101 to 104, 201 to 206, and 301 and 302 | element |
| 210 and 310 | graph |

**Claims**

1. A method for prediction executed by an information processing device, the method comprising:

   a step of training a neural network model based on actual data related to a prediction target; and
   a step of predicting an objective factor related to the prediction target based on a plurality of explanatory factors related to the prediction target by the neural network model, wherein
   the neural network model comprises an input layer, an intermediate layer, and an output layer, and a coefficient of an activation function related to the intermediate layer is larger than a coefficient of an activation function related to the output layer.

2. The method for prediction according to claim 1, wherein

   the activation functions related to the intermediate layer and the output layer are sigmoid functions determined by following Mathematical Formulas (1) and (2), respectively, and
   $\alpha_1$ and $\alpha_2$ satisfy $\alpha_1 > \alpha_2$:

   [Mathematical Formula 1]

   $$f^1\left(u_j^1\right) = \frac{1}{1+e^{-a_1 u_j^1}} \quad (1)$$

   $$f^2\left(u_j^2\right) = \frac{1}{1+e^{-a_2 u_j^2}} \quad (2)$$

   with proviso that $f^1\left(u_j^1\right)$ is the activation function related to the intermediate layer; $\alpha_1$ is the coefficient of the activation function related to the intermediate layer; $u_j^1$ is an input value input to a j-th of the intermediate layer; $f^2\left(u_j^2\right)$ is the activation function related to the output layer; $\alpha_2$ is the coefficient of the activation function related to the output layer; and $u_j^2$ is an input value input to a j-th of the output layer.

3. The method for prediction according to claim 1 or 2, wherein the prediction target includes a polycondensation reaction and an addition polymerization reaction.

4. The method for prediction according to claim 1 or 2, wherein number of elements in the intermediate layer is 1.1 times or more and less than 6 times of number of the explanatory factors.

5. The method for prediction according to claim 1 or 2, wherein a numerical value range of the explanatory factors input to the input layer is 0 or more and 1 or less and a numerical value range of the objective factor output from the output layer is 0.2 or more and 0.8 or less.

6. An information processing device comprising a control unit, wherein

the control unit

trains a neural network model based on actual data related to a prediction target, and
predicts an objective factor related to the prediction target based on a plurality of explanatory factors related to the prediction target by the neural network model, and

the neural network model comprises an input layer, an intermediate layer, and an output layer, and a coefficient of an activation function related to the intermediate layer is larger than a coefficient of an activation function related to the output layer.

7. A program executed by an information processing device, the program causing a computer to:

train a neural network model based on actual data related to a prediction target; and
predict an objective factor related to the prediction target based on a plurality of explanatory factors related to the prediction target by the neural network model, wherein
the neural network model comprises an input layer, an intermediate layer, and an output layer, and a coefficient of an activation function related to the intermediate layer is larger than a coefficient of an activation function related to the output layer.

FIG. 1

INFORMATION PROCESSING DEVICE — 10

CONTROL UNIT — 11

STORAGE UNIT — 12

INPUT UNIT — 13

OUTPUT UNIT — 14

FIG. 2

```
        ┌─────────────┐
        │    STRAT    │
        └──────┬──────┘
               │
               ▼
┌──────────────────────────────────────────┐
│  TRAIN NEURAL NETWORK MODEL BASED ON      │ ⌐S101
│  ACTUAL DATA RELATED TO CHEMICAL          │
│  REACTION OF SYNTHETIC RESIN              │
└──────────────────┬───────────────────────┘
                   │
                   ▼
┌──────────────────────────────────────────┐
│  PREDICT OBJECTIVE FACTOR RELATED TO      │ ⌐S102
│  CHEMICAL REACTION BASED ON PLURALITY     │
│  OF EXPLANATORY FACTORS RELATED TO        │
│  CHEMICAL REACTION                        │
└──────────────────┬───────────────────────┘
                   │
                   ▼
┌──────────────────────────────────────────┐
│  OUTPUT PREDICTION RESULT                 │ ⌐S103
└──────────────────┬───────────────────────┘
                   │
                   ▼
        ┌─────────────┐
        │     END     │
        └─────────────┘
```

EP 4 421 687 A1

100

101
102
103
104

EXPLANATORY
FACTORS

200
201
202
203
204
205
206

300
301
302

OBJECTIVE
FACTOR

LAYER FOR OBTAINING FEATURES OF
INPUT INFORMATION

LAYER FOR APPROXIMATING TO
VALUE OF OBJECTIVE FACTOR

VALUE OF SIGMOID FUNCTION

1.0
0.9
0.8
0.7
0.6
0.5
0.4
0.3
0.2
0.1
0.0

210

OUTPUT
(TO OUTPUT LAYER)

INPUT
(COMBINATION OF EXPLANATORY FACTORS)

COEFFICIENT

VALUE OF SIGMOID FUNCTION

1.0
0.9
0.8
0.7
0.6
0.5
0.4
0.3
0.2
0.1
0.0

310

OUTPUT
(TO OUTPUT LAYER)

INPUT
(COMBINATION OF EXPLANATORY FACTORS)

FIG. 3

FIG. 4

EP 4 421 687 A1

FIG. 5

FIG. 6

FIG. 7

## INTERNATIONAL SEARCH REPORT

| International application No. |
| --- |
| **PCT/JP2023/019011** |

**A. CLASSIFICATION OF SUBJECT MATTER**

*G06N 3/048*(2023.01)i; *C08L 101/00*(2006.01)i; *G06N 3/09*(2023.01)i; *G16C 20/70*(2019.01)i
FI: G06N3/048; C08L101/00; G06N3/09; G16C20/70

According to International Patent Classification (IPC) or to both national classification and IPC

**B. FIELDS SEARCHED**

Minimum documentation searched (classification system followed by classification symbols)
G06N3/048; C08L101/00; G06N3/09; G16C20/70

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2-287861 A (VICTOR CO OF JAPAN LTD) 27 November 1990 (1990-11-27) p. 3, lower left column, line 19 to p. 5, upper right column, line 7 | 1-7 |
| A | JP 4-266153 A (HONDA MOTOR CO LTD) 22 September 1992 (1992-09-22) paragraphs [0055]-[0095] | 1-7 |
| A | JP 11-232244 A (HITACHI LTD) 27 August 1999 (1999-08-27) paragraphs [0017]-[0081] | 1-7 |
| A | JP 9-091264 A (HITACHI MEDICAL CORP) 04 April 1997 (1997-04-04) paragraphs [0036]-[0038], fig. 6 | 1-7 |
| A | 岸田 悟 ほか, 階層型ニューラルネットワークのボタン選定問題への適用, 電子情報通信学会技術研究報告, 19 March 1993, vol. 92, no. 522, pp. 97-102, (KISHIDA, Satoru et al. Problems of Selecting the Buttons in the Layered Neural Network Application. IEICE Technical Report.) in particular, sections 3.4, 4 | 1-7 |

☑ Further documents are listed in the continuation of Box C.  ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **10 July 2023** | **18 July 2023** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

## INTERNATIONAL SEARCH REPORT

International application No.

**PCT/JP2023/019011**

### C.    DOCUMENTS CONSIDERED TO BE RELEVANT

| Category* | Citation of document, with indication, where appropriate, of the relevant passages | Relevant to claim No. |
|---|---|---|
| A | 渡邊 栄治 ほか, 講演における聴講者の動作の分析 (第3報), 電子情報通信学会技術研究報告, 17 January 2019, vol. 118, no. 420, LOIS2018-45, pp. 21-26, in particular, section 3.3, (WATANABE, Eiji et al. Analysis of behaviors of audience in presentations (Third report). IEICE Technical Report.)<br>in particular, section 3.3 | 1-7 |

Form PCT/ISA/210 (second sheet) (January 2015)

INTERNATIONAL SEARCH REPORT
Information on patent family members

International application No.

**PCT/JP2023/019011**

| Patent document cited in search report | | | Publication date (day/month/year) | Patent family member(s) | Publication date (day/month/year) |
|---|---|---|---|---|---|
| JP | 2-287861 | A | 27 November 1990 | EP 395112 A2 p. 3, line 33 to p. 7, line 47 | |
| JP | 4-266153 | A | 22 September 1992 | US 5280564 A column 6, line 55 to column 11, line 18 | |
| JP | 11-232244 | A | 27 August 1999 | (Family: none) | |
| JP | 9-091264 | A | 04 April 1997 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022212704 A

- WO 2003026791 A